# EUROPEAN PATENT SPECIFICATION

(11) **EP 2 351 762 B1**
(45) Date of publication and mention of the grant of the patent: **16.10.2013**
(21) Application number: 09821993.4
(22) Date of filing: 19.10.2009
(51) Int. Cl.: C07F 9/6512, C07D 405/06

(54) **NOVEL PYRIMIDINE DERIVATIVE AND METHOD FOR PRODUCING HMG-CoA REDUCTASE INHIBITOR INTERMEDIATE**
NEUES PYRIMIDINDERIVAT UND VERFAHREN ZUR HERSTELLUNG EINES ZWISCHENPRODUKTS EINES HMG-COA-REDUKTASEINHIBITORS
NOUVEAU DÉRIVÉ DE PYRIMIDINE ET MÉTHODE DE PRODUCTION D'UN INTERMÉDIAIRE D'INHIBITEUR DE HMG-CoA RÉDUCTASE

(30) Priority: 20.10.2008 JP 2008270076
(43) Date of publication of application: 03.08.2011
(73) Proprietor: KANEKA CORPORATION, Osaka (JP)
(72) Inventor: SAKA, Yasuhiro, Takasago-shi Hyogo 676-8688 (JP); NISHIYAMA, Akira, Takasago-shi Hyogo 676-8688 (JP)
(74) Representative: HOFFMANN EITLE
(86) International application number: PCT/JP2009/067987
(87) International publication number: WO 2010/047296

(56) References cited:
- WO-A1-2005/054207
- WO-A1-2007/017117
- JP-T- 2003 518 474

## Description

### TECHNICAL FIELD

The present invention relates to a novel pyrimidine derivative and method for producing an intermediate of an HMG-CoA reductase inhibitor.

### BACKGROUND ART

tert-Butyl (E)-(6-[2-[4-(4-fluorophenyl)-6-isopropyl-2-[methyl(methylsulfonyl)amino]pyrimidine-5-yl]vinyl](4R,6S)-2,2-dimethyl[1,3]dioxane-4-yl)acetate is an important intermediate of an HMG-CoA reductase inhibitor. As the production method thereof, the following methods have been known:
i) the method in which diphenyl[4-(4-fluorophenyl)-6-isopropyl-2-[methyl(methylsulfonyl)amino]pyrimidine-5-ylmethyl]phosphine oxide is reacted with tert-butyl 2-[(4R,6S)-6-formyl-2,2-dimethyl-1,3-dioxane-4-yl]acetate in the presence of sodium hexamethyldisilazide (Patent Document 1);
ii) the method in which triphenyl[4-(4-fluorophenyl)-6-isopropyl-2-[methyl(methylsulfonyl)amino]pyrimidine-5-ylmethyl]phosphonium bromide is reacted with tert-butyl 2-[(4R,6S)-6-formyl-2,2-dimethyl-1,3-dioxane-4-yl)acetate in the presence of potassium carbonate (Patent Document 2);
iii) the method in which 5-formyl-4-(4-fluorophenyl)-6-isopropyl-2-[methyl(methylsulfonyl)amino]pyrimidine is reacted with text-butyl 2-[(4R,6S)-6-[(dimethylphosphoryl)methyl]-2,2-dimethyl-1,3-dioxane-4-yl) acetate (Patent Document 3);
iv) the method in which triisopropyl [4-(4-fluorophenyl)-6-isopropyl-2-[methyl(methylsulfonyl)amino]pyrimidine-5-ylmethyl]phosphonium trifluoroacetate is reacted with methyl 3-formyl-(3S)-tert-butyldimethylsilyloxipropionate in the presence of a base, to synthesize methyl 5-(4-(4-fluorophenyl)-6-isopropyl-2-[methyl(methylsulfonyl)amino]pyrimidine-5-yl)-(3S)-tert-butyldimethylsilyloxy-4(E)-pentenoate, and then deriving the compound to tert-butyl (E)-(6-[2-[4-(4-fluorophenyl)-6-isopropyl-2-[methyl(methylsulfonyl)amino] pyrimidine-5-yl]vinyl](4R,6S)-2,2-dimethyl[1,3]dioxane-4-yl)acetate (Patent Document 4).

### PRIOR ART

### PATENT DOCUMENT

Patent Document 1: WO2000/04
Patent Document 2: WO2005/05420
Patent Document 3 : CN1687087
Patent Document 4 : WO2007/017117

### DISCLOSURE OF THE INVENTION

### PROBLEMS TO BE SOLVED BY THE INVENTION

However, inexpensively industrial production is difficult by the production method i), since it is needed to use expensive ethyldiphenylphosphinite. Concerning the production method ii), the property of triphenyl[4-(4-fluorophenyl)-6-isopropyl-2- [methyl(methylsulfonyl)amino]pyrimidine-5-ylmethyl]phosphonium bromide to be used as a solid is bad and the compound is not suitable for industrial use. The production method iii) is not also industrially-applicable, since it is needed to synthesize unstable tert-butyl 2-[(4R,6S)-6-[(dimethylphosphoryl)methyl]-2,2-dimethyl-1,3-dioxane-4-yl] acetate. The production method iv) is not suitable for inexpensively industrial production, since it is needed to use expensive trifluoroacetic acid in an equivalent amount and the carbon homologation reaction is repeated two times.

### MEANS FOR SOLVING THE PROBLEMS

The present inventors studied very hard for solving the problems. As a result, the present inventors found that a novel pyrimidine derivative such as dimethyl [4-(4-fluorophenyl)-6-isopropyl-2-[methyl (methylsulfonyl) amino]pyrimidine-5-ylmethyl]phosphonate can be produced from an inexpensive compound which is industrially available. In addition, the present inventors found that tert-butyl (E)-(6-[2-[4-(4-fluorophenyl)-6-isopropyl-2-[methyl(methylsulfonyl)amino] pyrimidine-5-yl]vinyl](4R,6S)-2,2-dimethyl[1,3]dioxane-4-yl)acetate, which is an important intermediate of an HMG-CoA reductase inhibitor, can be produced from the derivative synthesized by the present invention method, and completed the present invention.

The present invention relates to a method for producing a phosphonate ester represented by the formula (3), comprising the steps of
reacting a compound represented by the following formula (1) : 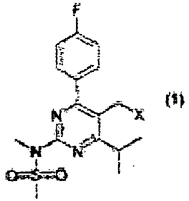 wherein, X is an elimination group,
with a compound represented by the following formula (2) :

(OR¹)₃P (2)

wherein, R¹ is Methyl or Ethyl to produce a phosphonate ester represented by the following formula (3): 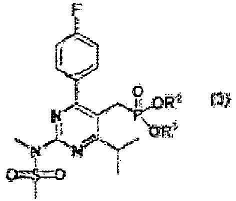 wherein, R¹is the same as the above,
and then obtaining a crystal of the compound (3) from the reaction mixture directly, without isolation.

The present invention also relates to a method for producing an intermediate of an HMG-CoA reductase inhibitor, comprising the step of
reacting the phosphonate ester represented by the formula (3) with a compound represented by the following formula (4): 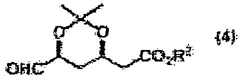 wherein, R² is an optionally substituted alkyl group having 1 to 18 carbon atoms, an optionally substituted aryl group having 6 to 18 carbon atoms, or an optionally substituted aralkyl group having 7 to 18 carbon atoms,
in the presence of a base,
wherein the intermediate is represented by the following formula (5): 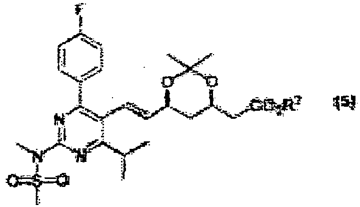 wherein, R² is the same as the above.

Furthermore, the present invention relates to a pyrimidine derivative represented by the following formula (3): 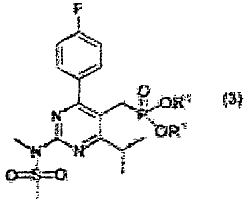 wherein, R¹ Methyl or Ethyl.

### EFFECT OF THE INVENTION

By the present invention method, a novel pyrimidine and tert-butyl (E)-(6-[2-[4-(4-fluorophenyl)-6-isopropyl-2-[methyl (methylsulfonyl)amino]pyrimidine-5-yl]vinyl] (4R,6S)-2,2-dimethyl[1,3]dioxane-4-yl)acetate, which is an important intermediate for an HMG-CoA reductase inhibitor, can be produced in industrially advantageous condition using inexpensive reagents.

### BRIEF DESCRIPTION OF DRAWING

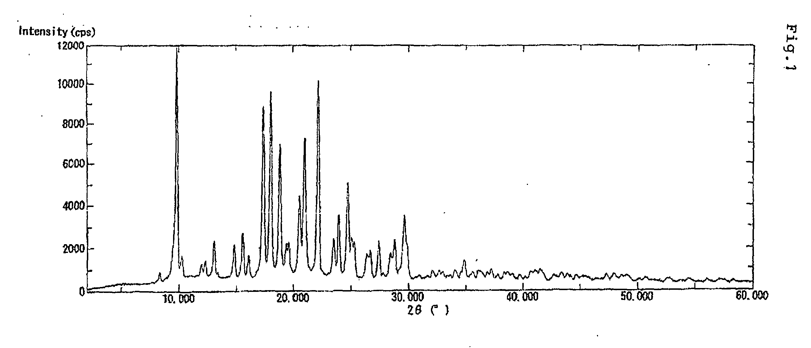

Figure 1 shows the X-ray powder analysis spectrum of the typical sample of dimethyl [4-(4-fluorophenyl)-6-isopropyl-2-[methyl (methylsulfonyl)amino]pyrimidine-5-ylmethyl]phosphonate. The vertical axis represents the intensity of X-ray (cps), and the horizontal axis represents diffraction angle (2θ).

### MODE FOR CARRYING OUT THE INVENTION

Hereinafter, the present invention is further described in detail.

The compound represented by the general formula (1): 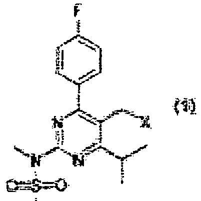 as a starting material can be produced by transforming the hydroxy group of 5-hydroxymethyl-4-(4-fluorophenyl)-6-isopropyl-2-[methyl(methylsulfonyl)amino]pyrimidine to an elimination group X.

The X is an elimination group. The X is exemplified by a halogen atom such as an iodine atom, a bromine atom and a chlorine atom; a sulfonate ester such as methanesulfonate, p-toluenesulfonate and trifluoromethanesulfonate; and others.

The hydroxy group can be transformed to a halogen atom by a general method, such as the method using thionyl chloride for the substitution to a chlorine atom, the method using phosphorus tribromide for the substitution to a bromine atom, and the method using iodine and triphenylphosphine for the substitution to an iodine atom.

The hydroxy group can be transformed to a sulfonate ester by a general method for synthesizing sulfonate ester, that is, by the method in which sulfonyl halide or sulfonic anhydride is reacted in the presence of a base. For example, 5-bromomethyl-4-(4-fluorophenyl)-6-isopropyl-2-[methyl(methylsulfonyl)amino]pyrimidine can be produced by the method of Reference Example 1.

A phosphonate ester represented by the formula (3): 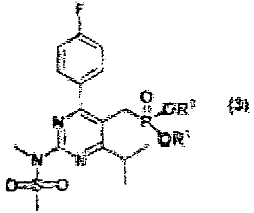 can be produced by reacting the compound represented by the formula (1) with the phosphite ester represented by a formula (2):

(OR¹)₃P (2)

R¹ for efficiently synthesizing the phosphonate ester at low cost is a methyl group and an ethyl group, especially a methyl group. The phosphite ester to be used is therefore preferably trimethyl phosphite and triethyl phosphite, more preferably trimethyl phosphite.

The use amount of phosphite ester is not particularly limited, and is generally within the range of 1 to 10 times by mole, preferably within the range of 1 to 5 times by mole, more preferably within the range of 1 to3 times by mole, relative to the mole of the compound represented by the formula (1). It is needed to use not less than 1 time by mole of phosphite ester for synthesizing the phosphonate ester represented by the formula (3) with high yield.

The reaction can be carried out in the absence of a solvent, or may be carried out in the presence of a solvent.

The solvent to be used is not particularly limited, but is exemplified by an aliphatic hydrocarbon solvent such as n-hexane, n-pentane, cyclohexane and methylcyclohexane; an aromatic hydrocarbon solvent such as toluene, ethylbenzene and o-dichlorobenzene; an acetate ester solvent such as methyl acetate, ethyl acetate, isopropyl acetate and n-butyl acetate; a nitrile solvent such as acetonitrile and propionitrile; an amide solvent such as N,N-dimethylformamide and N,N-dimethylacetamide; an ether solvent such as diethyl ether. tert-butyl methyl ether and tetrahydrofuran. Among the solvents, an aromatic hydrocarbon solvent is preferable, and toluene is especially preferable. One of the solvent may be used by itself, or plural solvents may be used in combination of optical ratio.

The use amount of the solvent is not particularly limited, but is generally 1 to 50 times by volume, preferably 1 to 20 times by volume, more preferably 1 to 10 times by volume, relative to the weight of the compound represented by the formula (1).

The temperature for the reaction is not particularly limited, but is generally within the range of 0 to 250°C, preferably within the range of 25 to 200°C, more preferably within the range of 50 to 200°C.

The reaction time is not particularly limited, but is generally within the range of 1 to 100 hours, preferably within the range of 1 to 72 hours, more preferably within the range of 1 to 48 hours.

The reaction is preferably carried out under an inert gas atmosphere, though the reaction may be carried out under untreated atmosphere. For example, the inert gas is preferably helium, nitrogen, argon and others, more preferably nitrogen and argon among the gases.

The above-mentioned reaction, in which the phosphonate ester is synthesized from the compound having an elimination group X and phosphite ester, has been known as Arbuzov reaction. In the reaction mechanism, as the following scheme 1, the substitutional reaction firstly occurs between the phosphorous atom of the phosphite ester represented by the formula (2) and the elimination group X of the compound represented by the formula (1), to generate the intermediate (A) ; and then the elimination group X attacks the substituent R¹ in the phophite ester so that the phosphonate ester represented (3) is generated and R¹-X is removed as a by-product.

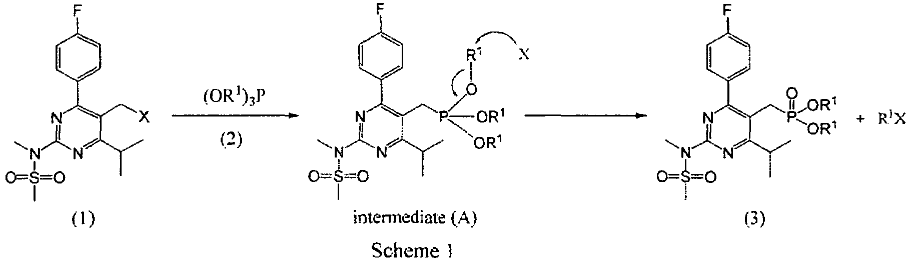

In the present invention method, the target compound (3) represented by the formula (3) can be directly isolated and purified as a crystal subsequent to the reaction from the reaction mixture without purification procedure such as silica gel column chromatography, which is difficult to be industrially carried out in terms of facilities and economy. In other words, the reaction solvent can be directly used as the solvent for crystallization.

An organic solvent which is different from the reaction solvent may be added for decreasing the solubility of the target phosphonate ester. Such a solvent to be added is not particularly limited, and is preferably an aliphatic hydrocarbon solvent such as n-hexane, n-pentane, cyclohexane and methylcyclohexane, more preferably hexane. It is deservingly possible that once the reaction mixture is concentrated and then different solvent is added to the reaction mixture for crystallization.

The method for the crystallization from the reaction mixture is not limited, and an appropriate usual method for crystallization may be used. For example, one of the following methods or plural following methods in combination can be used:
cooling crystallization method in which the reaction mixture solution is heated and then the solution is cooled for crystallization;
the method in which seed crystal is added in the reaction mixture for accelerating crystallization;
concentrating crystallization method in which the reaction mixture is concentrated to an appropriate concentration to be a supersaturated solution and then crystallization is carried out;
the method in which the solubility is decreased by adding a poor solvent for crystallization.

The temperature for crystallization is not particularly limited, but is generally within the range of -50 to 50°C, preferably within the range of -25 to 30°C, more preferably within the range of -15 to 15°C.

The crystallization can be carried out under untreated atmosphere, and can be carried out under an inert gas atmosphere subsequent to the reaction. As the inert gas, the same gases described in the reaction condition may be exemplified.

The mother liquid after crystallization contains excessively used phosphite ester in some cases; and such a mother liquid can be directly used for the next reaction.

The phosphite ester having a low boiling point, such as trimethyl phosphite having the boiling point of 111 to 112°C, can be separated from the reaction mixture by concentration and used again.

When the alkyl group of phosphite ester is a lower alkyl group such as a methyl group or an ethyl group and the X is a halogen atom, the boiling point of the by-product compound corresponding to a halogenated alkyl of R¹-X is low and the compound can be easily removed.

As mentioned above, by the method for producing the phosphonate ester represented by the formula (3) according to the present invention, a novel pyrimidine derivative and an HMG-CoA reductase inhibitor which is synthesized from the pyrimidine derivative can be obtained. In addition, the production method of the present invention is excellent in terms of industry and the pressure on the environment.

Next, the method for producing an intermediate of an HMG-CoA reductase inhibitor represented by the general formula (5) : 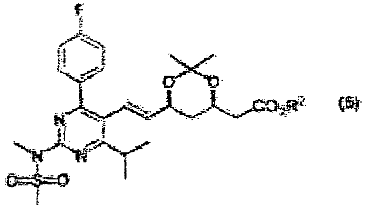 by reacting the compound represented by the formula (3) with a compound represented by the general formula (4): 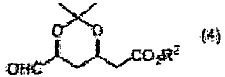 in the presence of a base, is described. The compound represented by the formula (3) may be obtained by the above-described method or the other methods.

The R² is an optionally substituted alkyl group having 1 to 18 carbon atoms, an optionally substituted aryl group having 6 to 18 carbon atoms, or an optionally substituted aralkyl group having 7 to 18 carbon atoms. The substituent can be exemplified by the same substituents for R¹.

As the optionally substituted alkyl group having 1 to 18 carbon atoms, a methyl group, an ethyl group, an n-propyl group, an isopropyl group, an n-butyl group, an isobutyl group, a sec-butyl group, a tert-butyl group, a pentyl group, a neopentyl group, a hexyl group, an octyl group, a nonyl group, a decyl group, an undecyl group, a dodecyl group, a tridecyl group, a tetradecyl group, a pentadecyl group, a hexadecyl group, a heptadecyl group, an octadecyl group and others can be exemplified.

As the optionally substituted aryl group having 6 to 18 carbon atoms, a phenyl group, a p-methoxyphenyl group, a p-chlorophenyl group, a naphthyl group and others can be exemplified.

As the optionally substituted aralkyl group having 7 to 18 carbon atoms, a benzyl group, a p-methoxybenzyl group, a naphthylmethyl group and others can be exemplified.

As the R², a tert-butyl group is preferable among the examples.

The use amount of the compound represented by the formula (4) is not particularly limited, but is generally within the range of 0.8 to 2.0 times by mole, preferably 1.0 to 1.5 times by mole, relative to the mole of the phosphonate ester represented by the formula (3).

The compound represented by the formula (4) can be the produced by, for example, the production method described in JP2573819B; the Swern oxidation method described in Tetrahedron Letters, vol.31, pp.2545-2548, 1990, in which tert-butyl 2-[(4R,6S)-6-hydroxymethyl-2,2-dimethyl-1,3-dioxane-4-yl)acetate is reacted with dimethylsulfoxide, triethylamine and oxalyl chloride; and the method described in Reference Example 2.

The reaction is carried out in the presence of a base. The kind of the base is not particularly limited, and an inorganic base or organic base can be used. As inorganic base, for example, an alkali metal hydroxide such as lithium hydroxide, sodium hydroxide and cesium hydroxide; an alkaline earth metal hydroxide such as magnesium hydroxide, calcium hydroxide and strontium hydroxide; a metal hydride such as lithium hydride, sodium hydride and calcium hydride; and others can be exemplified.

As organic base, for example, an amide base such as lithium diisopropylamide, lithium-2,2,6,6-tetramethylpiperazide, lithium hexamethyldisilazide, sodium hexamethyldisilazide and potassium hexamethyldisilazide; a Grignard reagent such as isopropylmagnesium bromide and tert-butylmagnesium chloride; an organic amine base such as 1,8-diazabicyclo[4.5.0]-7-undecene and 1,4-diazabicyclo[2.2.2]octane; and others can be exemplified.

Among the above examples, an amide base such as lithium diisopropylamide, lithium-2,2,6,6-tetramethylpiperazide, lithium hexamethyldisilazide, sodium hexamethyldisilazide and potassium hexamethyldisilazide is preferable, and lithium hexamethyldisilazide, sodium hexamethyldisilazide and potassium hexamethyldisilazide is more preferable.

The reaction is generally carried out under an inert atmosphere such as helium, nitrogen, neon and argon, preferably nitrogen and argon.

The use amount of the base is not particularly limited, but is generally within the range of 0.8 to 2.0 times by mole, preferably 1.0 to 1.5 times by mole, more preferably 1.0 to 1.3 times by mole, relative to the mole of the phosphonate ester represented by the formula (3).

The reaction is generally carried out in the presence of a solvent. It is necessary to select the solvent which is unreactive to a base, since the reaction is carried out in the presence of a base; however, such an unreactive solvent is not particularly limited as long as the solvent is unreactive to a base. One solvent may be used by itself, or plural solvents may be used in combination of optical ratio. As the solvent, an aliphatic hydrocarbon solvent such as n-hexane, n-pentane, cyclohexane and methylcyclohexane; an aromatic hydrocarbon solvent such as toluene, ethylbenzene and o-dichlorobenzene; an ether solvents such as diethyl ether, tert-butyl methyl ether and tetramethylfuran can be exemplified. Among the solvents, an aromatic hydrocarbon solvent and an ether solvent are preferable, and toluene and tetrahydrofuran are particularly preferable.

The use amount of the solvent is not particularly limited, but is generally within the range of 1 to 100 times by volume, preferably 5 to 60 times by volume, more preferably 5 to 50 times by volume, relative to the phosphonate ester represented by the formula (3) to be used.

The temperature for the reaction is not particularly limited as long as the temperature is not more than the boiling point of the usually-used solvent, but is preferably within the range of -100 to 100°C, more preferably within the range of -100 to 30°C, more preferably within the range of - 100 to 20°C.

The intermediate of an HMG-CoA reductase inhibitor which is represented by the formula (5) and synthesized by the above method can be easily isolated from the reaction mixture by a general procedure such as extraction. For example, the intermediate can be directly isolated from the reaction mixture and the purity thereof can be increased by crystallization.

Among the compound which is represented by the formula (3) and produced by the present invention, the pyrimidine derivative of which R¹ an optionally substituted alkyl group having 1 to 18 carbon atoms, an optionally substituted aryl group having 6 to 18 carbon atoms, or an optionally substituted aralkyl group having 7 to 18 carbon atoms has not been described in any documents and is a novel compound.

In particular, the pyrimidine derivative of which R¹ is a methyl group, i.e. dimethyl [4-(4-fluorophenyl)-6-isopropyl-2-[methyl(methylsulfonyl)amino]pyrimidine-5-ylmethyl]phosphonate, is a useful compound for synthesizing an intermediate of an HMG-CoA reductase inhibitor. Among the crystal forms of the compound, there is a crystal exhibiting an X-ray powder analysis spectrum having prominent ten peaks of 2θ = 9.8°, 17,4°, 18.0°, 18.8°, 20.5°, 20.9°, 22.1°, 23.9°, 24.7° and 29.6°.

The X-ray powder analysis spectrum was measured by evenly-filling the dimple of a slide glass with a crystal sample. The sample was scanned at a speed of 2.000°/min in the range of 2.000 to 60.000°. As the X-ray source, CuKα₁ ray was used; and the tube voltage and tube current were respectively set at 30 kV and 15 mA for measurement.

The X-ray powder analysis spectrum of the sample of the above pyrimidine derivative is shown as Figure 1. Needless to say, the values in the Figure should not be regarded as absolute, since 2θ value in X-ray powder analysis pattern can be slightly different depending on a device or sample in some cases. In addition, when the identity of crystal is judged, the whole diffraction pattern is important in x-ray powder analysis pattern due to the nature of data.

### EXAMPLES

The present invention is hereinafter explained in more detail with examples; however, the present invention is not limited by the examples.

### Reference Example 1: 5-Bromomethyl-4-(4-fluorophenyl)-6-isopropyl-2- [methyl (methylsulfonyl) amino]pyrimidine

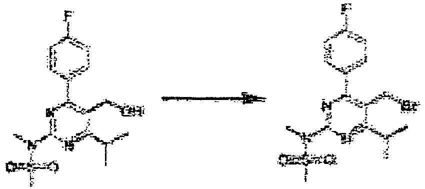

To the mixed solution of 5-hydroxymethyl-4-(4-fluorophenyl)-6-isopropyl-2-[methyl(methylsulfonyl)amino] pyrimidine (15 g, 42.42 mmol) dissolved in anhydrous toluene (120 ml) and anhydrous acetonitrile (60 ml), phosphorus tribromide (5.4 g, 19.44 mmol) was slowly added dropwise at 10 to 20°C under nitrogen stream. After the reaction for 1 hour, saturated brine (100 ml) and distilled water (10 ml) were added to the reaction mixture, and the mixture was stirred for 10 minutes. After the aqueous layer was separated and discarded, the organic layer was sequentially washed with a saturated sodium hydrogencarbonate aqueous solution (50 ml) and distilled water (50 ml). The organic layer was dried with anhydrous magnesium sulfate, and the solvent was distilled away under reduced pressure. The obtained crude product was dispersed in hexane (100 ml), and the title compound (17.5 g, yield: 99%) was obtained as a white solid by filtration. ¹H-NMR(CDCl₃): δ1.36H, d, J=6.6Hz), 3.48(1H, m), 3.51(3H, s), 3.56 (3H, s), 4.48(2H, s), 7.20 (2H, m), 7.80(2H, m)

### Reference Example 2: tert-Butyl 2-[(4R,6S)-6-formyl-2,2-dimethyl-1,3-dioxane-4-yl]acetate

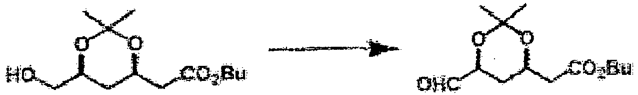

To a dispersion of tert-butyl 2-[(4R,6S)-6-hydroxymethyl-2,2-dimethyl-1,3-dioxane-4-yl]acetate (15 g, 57.6 mmol), sodium hydrogencarbonate (13.6 g, 161.3 mmol), potassium bromide (1.37 g, 11.5 mmol) and 4-hydroxy-2, 2, 6, 6-tetramethylpiperidine-1-oxyl free radical (248 mg, 1.44 mol) in ethyl acetate (150 ml), an aqueous solution of sodium hypochlorite (44.2 g, 16.7wt%, 70.2 mmol) was added under nitrogen stream at -10°C carefully dropwise to keep the inner temperature within 5°C. After the dropwise addition, the mixture was stirred at 0°C for 1 hour and the aqueous layer was separated. The organic layer was further diluted with ethyl acetate (100 ml), and sequentially washed with a 5% sodium thiosulfate aqueous solution (75 ml) and water (40 ml × 2). Then, the organic layer was dried with anhydrous magnesium sulfate. The solvent was distilled away under reduced pressure, and the obtained crude product was purified with silica gel column chromatography (silica gel: 200 g, developing solvent: hexane / ethyl acetate = 2 / 1 by volume) to obtain the title compound (8.0 g, yield: 54%) as white crystals.
¹H-NMR(CDCl₃): δ1.35(1H, q, J=12.7Hz), 1.45(9H, s), 1.46(3H, s), 1.49(3H, s), 1.83(1H, dt, J=2.7Hz, 12.9Hz), 2.35(1H, dd, J=5.9Hz, 15.4Hz), 2.46(1H, dd, J=7.1Hz, 15.4Hz), 4.33(1H, m), 9.58(1H, s)

### Example 1: Dimethyl [4-(4-fluorophenyl)-6-isopropyl-2-[methyl(methylsulfonyl)amino]pyrimidine-5-ylmethyl]phosphonate

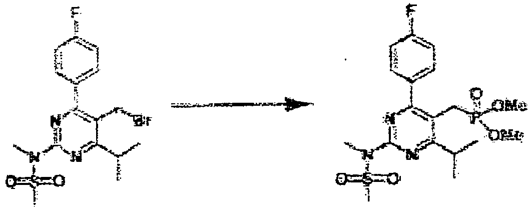

To a solution of 5-bromomethyl-4-(4-fluorophenyl)-6-isopropyl-2-[methyl(methylsulfonyl)amino]pyrimidine (10.0 g, 24.02 mmol) produced in Reference Example 1 dissolved in anhydrous toluene (50 ml), trimethyl phosphite (7.5 g, 60.05 mmol) was added at room temperature under nitrogen stream. After the reaction mixture was heated to reflux for 18 hours, the solvent was distilled away under reduced pressure. The procedure in which toluene (50 ml) was added to the concentrated residue and the mixture was concentrated was repeated two times. The residue was dissolved in toluene (20 ml). After hexane (17 ml) was slowly added thereto dropwise, previously-prepared seed crystal of the title compound (about 20 mg) was added thereto for crystallization. The mixture was aged at room temperature for 30 minutes, and then hexane (13 ml) was slowly added dropwise. The mixture was cooled to 0°C. After the mixture was aged for 30 minutes, the precipitated crystals were separated. After the crystals were sequentially washed with toluene / hexane = 4 / 1 by volume (10 ml), then toluene / hexane = 4 / 1 by volume (15 ml), further hexane (20 ml), and then dried at 40°C under reduced pressure to obtain the title compound (10.6 g, yield : 99%) as a white solid. The compound was analyzed with ¹H-NMR; as a result, impurity could not be observed.
¹H-NMR(CDCl₃) : δ1.30(6H, d, J=6.6Hz), 3.30(2H, d, J=22.0Hz), 3.44(1H, quint, J=6.6Hz), 3.49(3H, s), 3.54(3H, s), 3.55(3H, s), 3.58(3H, s), 7.17(2H, m), 7.59(2H, m)

Example 2: tert-Butyl (E)-(6-[2-[4-(4-fluorophenyl)-6-isopropyl -2-[methyl(methylsulfonyl)amino]pyrimidine-5-yl]vinyl(4R,6S)-2,2-dimethyl[1,3]dioxane-4-yl)acetate

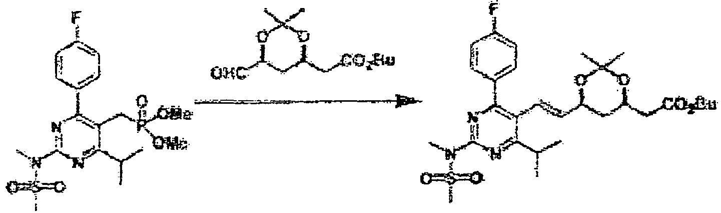

To a solution of dimethyl [4-(4-fluorophenyl)-6-isopropyl-2-[methyl(methylsulfonyl)amino]pyrimidine-5-ylmethyl] phosphonate (5.0 g, 11.22 mmol) produced in Example 1 dissolved in anhydrous THF (30 ml), a 1.9 M tetrahydrofuran solution of sodium hexamethyldisilazide (6.8 ml, 12.91 mmol) was added dropwise at -78°C for 10 minutes under nitrogen stream. After the mixture was aged at -78°C for 20 minutes, a solution of tert-butyl 2-[(4R,6S)-6-formyl-2,2-dimethyl-1,3-dioxane-4-yl]acetate (3.62 g, 14.03 mmol) dissolved in anhydrous tetrahydrofuran (10.9 ml) was added dropwise for 20 minutes. The device for dropwise addition was washed with anhydrous tetrahydrofuran (2.3 ml), and the wash fluid was added to the reaction mixture dropwise. The temperature of the reaction mixture was raised to -35°C for 2 hours, and the reaction was carried out at the same temperature for 14 hours. The temperature was raised to 0°C for 1 hour, and the mixture was aged for 1 hour. The reaction was terminated by adding a saturated ammonium chloride aqueous solution (50 ml) to the reaction mixture, and then the mixture was extracted with toluene (100 ml). The organic layer was washed with distilled water (25 ml) two times and saturated brine (20 ml) two times. The quantity of the content amount of tert-butyl (E)-(6-[2-[4-(4-fluorophenyl)-6-isopropyl-2-[methyl(methylsulfonyl)amino] pyrimidine-5-yl]vinyl](4R,6S)-2,2-dimethyl[1,3]dioxane-4-yl)acetate in the organic layer was determined with high-performance liquid chromatography; as a result, the title compound (5.5 g, yield: 85%) was contained.

After the organic solvent was distilled away under reduced pressure, methanol (40 ml) was added thereto and the mixture was heated to 55°C. The solution was cooled to 45°C, and then previously-prepared seed crystal of the title compound (about 10 mg) was added thereto for crystallization. After the start of crystallization was observed, the mixture was cooled to room temperature. The mixture was further cooled to -10°C, and aged for 30 minutes. The precipitated crystals were separated filtration, and the obtained cake was washed with methanol (15 ml) at -10°C. The crystals were dried at 40°C under reduced pressure, to obtain the title compound (3.9 g).
¹H-NMR(CDCl₃): δ1.13(1H, q, J=12.7Hz), 1.26(3H, d, J=6.8Hz), 1.27(3H, d, J=6.8Hz), 1.40(3H, s), 1.46(9H, s), 1.49(3H, s), 1.54(1H, dt, J=2.5Hz, 12.7Hz), 2.30(1H, dd, J=6.3Hz, 15.4Hz), 2.45(1H, dd, J=6.8Hz, 15.4Hz), 3.38(1H, sep, J=6.8Hz), 3.52(3H, s), 3.57(3H, s), 4.28(1H, m), 4.43(1H, m), 5. 46 (1H, dd, J=5.4Hz, 16.4Hz), 6.51(1H, dd, J=1.2Hz, 16.4Hz), 7.08 (2H, m), 7.64(2H,m)

Example 3: tert-Butyl (E)-(6-[2-[4-(4-fluorophenyl)-6-isopropyl-2-[methyl(methylsulfonyl)amino]pyrimidine-5-yl]vinyl](4R,6S)-2,2-dimethyl[1,3]dioxane-4-yl)acetate

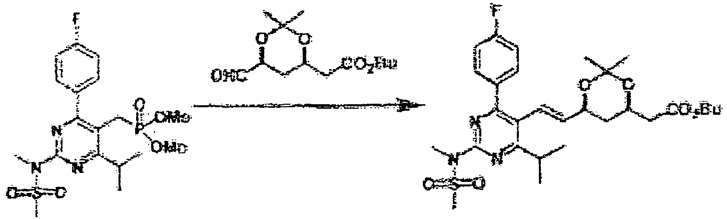

To a solution of dimethyl [4-(4-fluorophenyl)-6-isopropyl-2-[methyl(methylsulfonyl)amino]pyrimidine-5-ylmethyl] phosphonate (1.0 g, 2.25 mmol) produced in Example 1 dissolved in anhydrous tetrahydrofuran (20 ml), a 1.9 M tetrahydrofuran solution of sodium hexamethyldisilazide (1.4 ml, 2.58 mmol) was added dropwise at -78°C under nitrogen stream for 5 minutes. After the mixture was aged at -78°C for 15 minutes, a solution of tert-butyl 2-[(4R,6S)-6-formyl-2,2-dimethyl-1,3-dioxane-4-yl]acetate (0.7 g, 2.69 mmol) dissolved in anhydrous tetrahydrofuran (7 ml) was added thereto dropwise for 10 minutes. The device for dropwise addition was washed with anhydrous tetrahydrofuran (2 ml), and the wash fluid was added to the reaction mixture dropwise. The temperature of the reaction mixture was raised to -35°C for 3 hours, and the reaction was carried out at the same temperature for 15 hours. The temperature was raised to 0°C for 2 hour, and the mixture was aged for 1 hour. The reaction was terminated by adding a saturated ammonium chloride aqueous solution (20 ml) to the reaction mixture, and then the mixture was extracted with toluene (50 ml). The organic layer was washed distilled water (10 ml) two times and saturated brine (10 ml) two times. The quantity of the content amount of tert-butyl (E)-(6-[2-[4-(4-fluorophenyl)-6-isopropyl-2-[methyl(methylsulfonyl)amino] pyrimidine-5-yl]vinyl](4R,6S)-2,2-dimethyl[1,3]dioxane-4-yl)acetate in the organic layer was determined with high-performance liquid chromatography; as a result, the title compound z.2 g, yield: 95%) was contained.

### Example 4

The X-ray powder analysis spectrum of dimethyl [4-(4-fluorophenyl)-6-isopropyl-2-[methyl(methylsulfonyl)amino] pyrimidine-5-ylmethyl]phosphonate produced in Example 1 was shown as Figure 1. Ten remarkable peaks of 2θ = 9.8°, 17.4°, 18.0°, 18.8°, 20.5°, 20.9°, 22.1°, 23.9°, 24.7° and 29.6° was observed in XRD.
X-ray powder diffraction device: MiniFlex-II manufactured by Rigaku corporation
Condition: using CuKα₁ radiation
Tube voltage: 30kV
Emitter to base voltage: 15mA

## Claims

1. A method for producing a phosphonate ester comprising the steps of:
reacting a compound represented by the following formula (1): 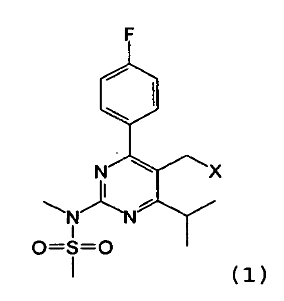 wherein X is an elimination group,
with a compound represented by the following formula (2) :
(OR¹)₃P (2)
wherein R¹ is selected from a methyl group or an ethyl group, to produce a phosphonate ester represented by the following formula (3): 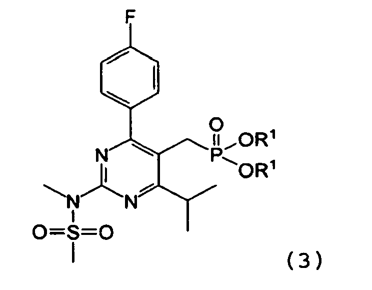 wherein R¹ is the same as defined above,
and then obtaining a crystal of the compound (3) from the reaction mixture directly without isolation.

2. A method for producing an intermediate represented by formula (5) of an HMG-CoA reductase inhibitor comprising reacting, in the presence of a base, a phosphonate ester represented by formula (3) with a compound represented by formula (4): 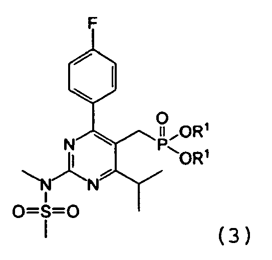 wherein R¹ is selected from a methyl group or an ethyl group; 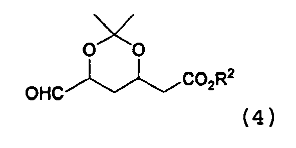 wherein R² is an optionally substituted alkyl group having 1 to 18 carbon atoms, an optionally substituted aryl group having 6 to 18 carbon atoms, or an optionally substituted aralkyl group having 7 to 18 carbon atoms; 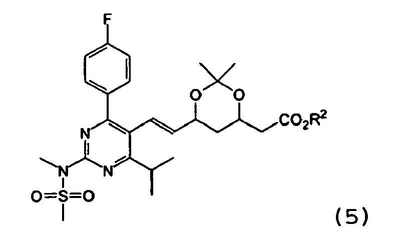 wherein R² is the same as defined above.

3. A method according to Claim 2, wherein the phosphonate ester represented by formula (3) is produced in accordance with the method as defined in Claim 1.

4. A method according to any preceding Claim, wherein X is a chlorine atom, a bromine atom or an iodine atom.

5. A method according to any preceding Claim, wherein R¹ is a methyl group and R² is a tert-butyl group.

6. A pyrimidine derivative represented by formula (3): 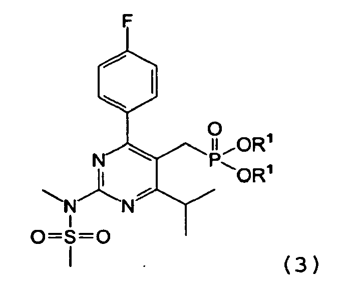 wherein R¹ is selected from a methyl group or an ethyl group.

7. A pyrimidine derivative according to Claim 6, wherein R¹ is a methyl group.

8. A pyrimidine derivative according to Claim 7 which exhibits an x-ray powder analysis pattern having specific peaks of 2θ = 9.8°, 17.4°, 18.0°, 18.8°, 20.5°, 20.9°, 22.1°, 23.9°, 24.7° and 29.6°.

## Patentansprüche

1. Verfahren zum Herstellen eines Phosphonatesters umfassend die Schritte:
Umsetzen einer Verbindung der folgenden Formel (1) 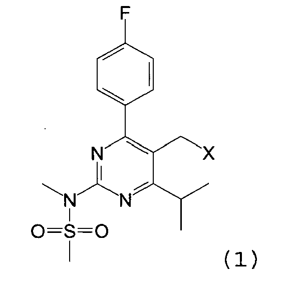 worin X eine Abgangsgruppe darstellt,
mit einer Verbindung der folgenden Formel (2):
(OR¹)₃P (2)
worin R¹ ausgewählt ist aus einer Methylgruppe oder einer Ethylgruppe, um einen durch die folgende Formel (3) dargestellten Phosphonatester herzustellen, 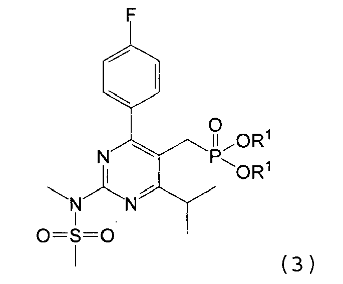 worin R¹ wie oben definiert ist,
und anschließend direktes Erhalten eines Kristalls, der Verbindung (3) aus der Reaktionsmischung ohne Isolieren.

2. Verfahren zum Herstellen eines durch die Formel (5) dargestellten Intermediats eines
HMG-CoA-Reduktaseinhibitors umfassend das Umsetzen eines Phosphonatesters der Formel (3) mit einer Verbindung der
Formel (4) in Gegenwart einer Base, 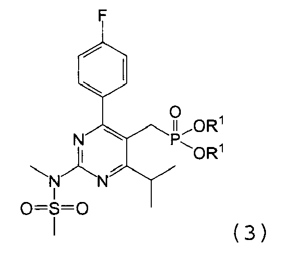 worin R¹ ausgewählt ist aus einer Methylgruppe oder einer Ethylgruppe, 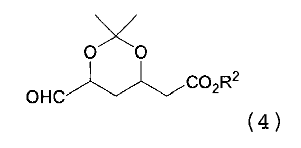 worin R² eine gegebenenfalls substituierte Alkylkguppe mit 1 bis 18 Kohlenstoffatomen, eine gegebenenfalls substituierte Arylgruppe mit 6 bis 18 Kohlenstoffatomen, oder eine gegebenenfalls substituierte Aralkylgruppe mit 7 bis 18 Kohlenstoffatomen, ist, 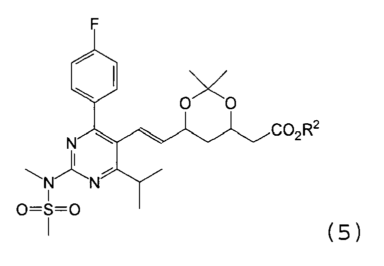 worin R² wie oben definiert ist.

3. Verfahren gemäß Anspruch 2, worin der Phosphonatester der Formel (3) gemäß dem in Anspruch 1 definierten Verfahren hergestellt wird.

4. Verfahren gemäß einem vorsehenden Anspruch, worin X ein Chloratom, ein Bromatom oder ein Iodatom ist.

5. Verfahren gemäß einem vorstehenden Anspruch, worin R¹ eine Methylgruppe und R² eine tert-Butylgruppe ist.

6. Pyrimidinderivat der Formel (3): 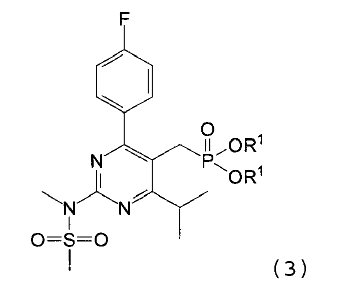 worin R¹ ausgewählt ist aus einer Methylgruppe oder einer Ethylgruppe.

7. Pyrimidinderivat gemäß Anspruch 6, worin R¹ eine Methylgruppe ist.

8. Pyrimidinderivat gemäß Anspruch 7, das ein Röntgen-Pulveranalysemuster mit spezifischen Peaks bei 2θ = 9,8°, 17,4°, 18,0°, 18,8°, 20,5°, 20,9°, 22,1°, 23,9°, 24,7° und 29,6° zeigt.

## Revendications

1. Procédé de production d'un ester de phosphonate, comprenant les étapes de :
réaction d'un composé représenté par la formule (1) suivante : 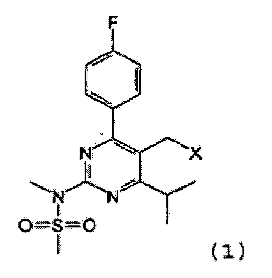 dans laquelle, X est un groupe d'élimination,
avec un composé représenté par la formule (2) suivante :
(OR¹)₃P (2)
dans laquelle, R¹ est un groupe choisi parmi un groupe méthyle ou un groupe éthyle, pour produire un ester de phosphonate représenté par la formule (3) suivante : 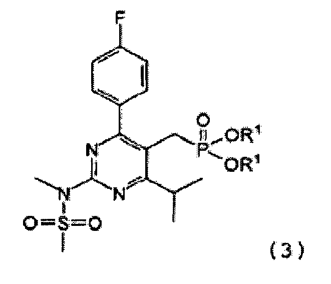 dans laquelle, R¹ est le même que celui défini ci-dessus,
puis l'obtention d'un cristal du composé (3) à partir du mélange réactionnel, directement sans isolement.

2. Procédé de production d'un intermédiaire représenté par la formule (5) d'un inhibiteur de l'HMG-CoA réductase comprenant la réaction, en présence d'une base, d'un ester de phosphonate représenté par la formule (3) avec un composé représenté par la formule (4) : 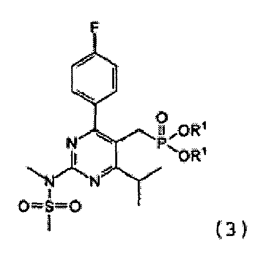 dans laquelle R¹ est choisi parmi un groupe méthyle ou
un groupe éthyle ; 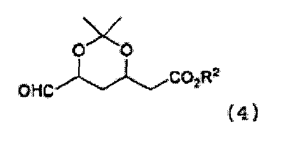 dans laquelle, R² est un groupe alkyle éventuellement substitué ayant 1 à 18 atomes de carbone, un groupe aryle éventuellement substitué ayant 6 à 18 atomes de carbone, ou un groupe aralkyle éventuellement substitué ayant 7 à 18 atomes de carbone ; 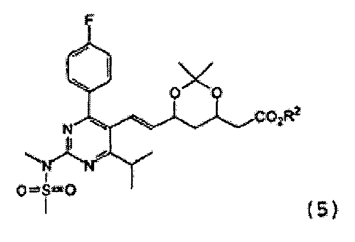 dans laquelle, R² est le même que celui défini ci-dessus.

3. Procédé selon la revendication 2, dans lequel l'ester de phosphonate représenté par la formule (3) est produit conformément au procédé selon la revendication 1.

4. Procédé selon n'importe quelle revendication précédente, dans lequel X est un atome de chlore, un atome de brome ou un atome d'iode.

5. Procédé selon n'importe quelle revendication précédente, dans lequel R¹ est un groupe méthyle et R² est un groupe tert-butyle.

6. Dérivé pyrimidine représenté par la formule (3) : 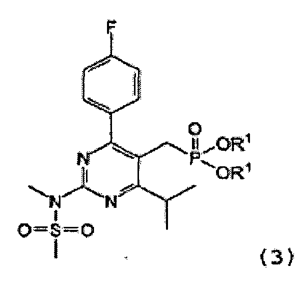 dans laquelle R¹ est choisi parmi un groupe méthyle ou un groupe éthyle.

7. Dérivé pyrimidine selon la revendication 6, dans lequel R¹ est un groupe méthyle.

8. Dérivé pyrimidine selon la revendication 7, qui présente un diagramme d'analyse par rayons X sur poudre ayant des pics spécifiques de 2θ = 9,8°, 17,4°, 18,0°, 18,8°, 20,5°, 20,9°, 22,1°, 23,9°, 24,7° et 29,6°.
